# EUROPEAN PATENT APPLICATION

(11) **EP 2 837 938 A1**
(43) Date of publication of application: **18.02.2015**
(21) Application number: 13770337.7
(22) Date of filing: 29.03.2013
(51) Int. Cl.: G01N 33/53, G01N 33/543, C07K 14/47

(54) **C1q-ADIPONECTIN COMPLEX AND USE THEREOF**

(30) Priority: 30.03.2012 JP 2012078540
(71) Applicant: Osaka University, Suita-shi, Osaka 565-0871 (JP); Otsuka Pharmaceutical Co., Limited, Tokyo 101-8535 (JP)
(72) Inventor: SHIMOMURA, Iichiro, Suita-shi Osaka 565-0871 (JP); FUNAHASHI, Tohru, Suita-shi Osaka 565-0871 (JP); KISHIDA, Ken, Suita-shi Osaka 565-0871 (JP); KOBAYASHI, Hironori, Osaka-shi Osaka 5400021 (JP); TAKAHASHI, Shigeo, Osaka-shi Osaka 5400021 (JP)
(74) Representative: Hoffmann Eitle
(86) International application number: PCT/JP2013/059467
(87) International publication number: WO 2013/147123

(57) **Abstract**

An object of the present invention is to provide a biomarker that can be used as a precise diagnostic marker for MetS or arteriosclerosis risk.

The present invention provides a C1q-adiponectin complex comprising naturally occurring adiponectin and Clq.

## Description

### Technical Field

The present invention relates to a C1q-adiponectin complex (also abbreviated as "C1q-APN" in the present specification) and use thereof. Moreover, the present invention relates to a method for measuring the amount of C1q-adiponectin complexes, and a method for diagnosing metabolic syndrome or arteriosclerosis risk by measuring the amount of the complexes.

### Background Art

Patients with metabolic syndrome (also abbreviated as "MetS" in the present specification) are considered to be at high risk for arteriosclerosis and life-threatening diseases, such as heart disease and stroke. Therefore, appropriate methods for diagnosing MetS are desired for prevention or treatment thereof.

Primary pathological conditions of MetS are insulin resistance and abnormal glucose tolerance, lipoprotein abnormalities which causes atherosclerosis, hypertension, and other symptoms caused by visceral fat accumulation. MetS is defined as a combination of visceral fat accumulation and two or more pathological conditions. Diagnostic criteria for MetS were created in April 2005, by eight societies in the field of internal medicine in Japan. Specifically, a person who has visceral fat obesity, and also has two or more of hypertension, abnormal lipid metabolism, and fasting hyperglycemia is diagnosed as MetS (NPL 1).

In recent years, various measurement methods for evaluating the degree of arteriosclerotic lesions have been developed, and their availability has been demonstrated. Typical examples of such diagnostic methods include:
(1) carotid artery ultrasonography that measures carotid intima-media thickness (IMT) by carotid ultrasound; and
(2) PWV (pulse wave velocity) or CAVI (cardio-ankle vascular index) test that measures blood pressure or pulse wave velocity between arm and ankle thereby numerically express the degree of arteriosclerosis.

In addition to these diagnostic methods, a method for measuring the total amount of adiponectin (also abbreviated as "Total-APN" in the present specification), or a method for measuring the amount of high-molecular-weight adiponectin (also abbreviated as "HMW-APN" in the present specification) (PTL 1) has been developed as a blood indicator of MetS associated with visceral fat accumulation, or arteriosclerotic disease.

On the other hand, as described later, basic research and clinical research results are accumulated that indicate direct connection of the reduction in blood levels of adiponectin (also abbreviated as "APN" in the present specification) associated with visceral fat accumulation, to cardiovascular diseases, as well as to diabetes, hyperlipidemia, and hypertension. For example, clinical research and basic research report that adiponectin has anti-diabetic action and anti-arteriosclerosis action (NPL 2). In particular, the reduction in blood adiponectin levels associated with obesity is called hypoadiponectinemia, which is revealed to be deeply involved in various obesity-related diseases, such as diabetes (NPL 3), hypertension (NPL 4), sleep apnea-hypopnea syndrome (NPL 5), arteriosclerotic cardiovascular disease (NPL 6), and metabolic syndrome (NPL 7).

Therefore, the measurement of blood adiponectin levels is expected to be able to become a diagnostic marker for MetS.

NPL 8 discloses a complex comprising C1q and adiponectin chemically treated with an oxidant (sodium metaperiodate); however, it has not been known until now that adiponectin and C1q form a complex in a natural state.

### Citation List

### Patent Literature

PTL 1: WO2009/078151

### Non-patent Literature

NPL 1: Metabolic syndrome diagnostic criteria research committee: Definition and diagnostic criteria for metabolic syndrome, The Journal of the Japanese Society of Internal Medicine, 94: 794-809, 2005
NPL 2: Matsuzawa Y, Adiponectin: a key player in obesity related disorders, Curr Pharm Des, 2010; 16: pp. 1896-901
NPL 3: Hotta K, Funahashi T, Arita Y, et al., Plasma concentrations of a novel, adipose-specific protein, adiponectin, in type 2 diabetic patients, Arterioscler Thromb Vasc Biol, 2000; 20: pp. 1595-9
NPL 4: Ohashi K, Kihara S, Ouchi N, et al., Adiponectin replenishment ameliorates obesity-related hypertension, Hypertension, 2006; 47: pp. 1108-16
NPL 5: Nakagawa Y, Kishida K, Kihara S, et al., Nocturnal reduction in circulating adiponectin concentrations related to hypoxic stress in severe obstructive sleep apnea-hypopnea syndrome, Am J Physiol Endocrinol Metab, 2008; 294: pp. E778-84
NPL 6: Ouchi N, Kihara S, Arita Y, et al., Novel modulator for endothelial adhesion molecules: adipocyte-derived plasma protein adiponectin, Circulation, 1999; 100: pp. 2473-6
NPL 7: Ryo M, Nakamura T, Kihara S, et al., Adiponectin as a biomarker of the metabolic syndrome, Circ J. 2004; 68: pp. 975-81.
NPL 8: Peake PW, et al., Adiponectin binds C1q and activates the classical pathway of complement, Biochem Biophys Res Commun, 367: 560-565, 2008

### Summary of Invention

### Technical Problem

As described above, adiponectin (also abbreviated as "APN" in the present specification) is a biomarker expected to serve as a diagnostic marker for MetS or arteriosclerotic disease. As adiponectin measurement methods, a method for measuring the Total-APN amount and a method for measuring the HMW-APN amount have been developed.

However, these measurement methods are not sufficiently accurate under the present circumstances, and more precise markers are required.

The measurement of the Total-APN amount cannot detect qualitative changes of adiponectin, because three-dimensional structures are dissociated by heat-treatment in the presence of SDS. Moreover, the measurement of the HMW-APN amount can detect hexamers or higher-order multimers of adiponectin; however, there is no specificity in detectable multimeric structures.

Meanwhile, in adiponectin-related measurement methods, there is a supposed possibility that detection of qualitative changes in addition to quantitative changes, is important. If the presence form of adiponectin complexes in the blood can be clarified, and a detection system specific to adiponectin complexes in the blood can be developed, it will be possible to diagnose MetS associated with visceral fat accumulation or a high-risk group of arteriosclerotic disease more efficiently than a method for measuring adiponectin alone.

Therefore, an object of the present invention is to provide a biomarker that can be used as a precise diagnostic marker for MetS or arteriosclerosis risk.

### Solution to Problem

In recent years, it has been suggested that adiponectin has the potential to directly bind to other proteins. *In vitro* analysis shows that adiponectin binds to proteins or glycolipids, such as complement C1q (NPL 8), Factor H (NPL 9), platelet-derived growth factor (PDGF)-BB (NPL 10), calreticulin (NPL 11), cystatin C (NPL 12), and lipopolysaccharide (NPL 13). However, it has not been analyzed that endogenous adiponectin present in human blood binds to these factors, and it has been unclear whether blood adiponectin forms complexes with other proteins.

Meanwhile, studies mainly conducted by Osaka University report the following:
(1) human adipose tissue abundantly expresses genes involved with the classical and alternative pathways of complement activation (NPL 14);
(2) adiponectin has high homology with C1q and binds to C1q receptors (NPL 15); and
(3) analysis using mouse arthritis models shows that adiponectin suppresses inflammation or joint destruction by inhibiting C1q or C3 (NPL 16).

In the present invention, the present inventors proceeded to search for adiponectin-binding proteins by far-Western blotting and *in vitro* interaction analysis using a protein-immobilized plate, and found strong binding of C1q to adiponectin. Further, the present inventors demonstrated, for the first time in the world, direct binding of C1q to endogenous adiponectin in human serum by co-immunoprecipitation using an anti-human adiponectin antibody and an anti-human C1q antibody.

NPL 9: Peake PW et al., Factor H binds to the N-terminus of adiponectin and modulates complement activation, Biochem Biophys Res Commun, 397: 361-366, 2010
NPL 10: Arita Y, Kihara S, Ouchi N, et al., Adipocyte-derived plasma protein adiponectin acts as a platelet-derived growth factor-BB-binding protein and regulates growth factor-induced common postreceptor signal in vascular smooth muscle cell, Circulation, 2002; 105: 2893-8
NPL 11: Takemura Y, Ouchi N, Shibata R, et al. Adiponectin modulates inflammatory reactions via calreticulin receptor-dependent clearance of early apoptotic bodies, J Clin Invest, 2007; 117: 375-86
NPL 12: Komura N, Kihara S, Sonoda M, et al., Increment and impairment of adiponectin in renal failure, Cardiovasc Res, 2010; 86: 471-7
NPL 13: Peake PW, et al., Human adiponectin binds to bacterial lipopolysaccharide, Biochem Biophys Res Commun, 341: 108-115, 2006
NPL 14: Maeda K, Okubo K, Shimomura I, et al., Analysis of an expression profile of genes in the human adipose tissue, Gene, 1997; 190: 227-35
NPL 15: Yokota T, Oritani K, Takahashi I, et al., Adiponectin, a new member of the family of soluble defense collagens, negatively regulates the growth of myelomonocytic progenitors and the functions of macrophages, Blood, 2000; 96: 1723-32
NPL 16: Ebina K, Oshima K, Matsuda M, et al., Adenovirus-mediated gene transfer of adiponectin reduces the severity of collagen-induced arthritis in mice, Biochem Biophys Res Commun, 2009; 378: 186-91

Next, for the purpose of clarifying clinical significance, the present inventors developed a sandwich ELISA method that can easily measure C1q-adiponectin complexes in human serum. C1q is known to have the physiological function of binding to the Fc region of an antibody to activate the complement pathway. This physiological function caused a non-specific reaction, and made it difficult to establish an ELISA method for measuring C1q-adiponectin complexes. In order to solve this problem, an F(ab')2 antibody lacking the Fc region was produced and used as the immobilized antibody, thereby realizing removal of the non-specific reaction. As a result of considering the above conditions, an ELISA method has been completed that can easily, specifically, and accurately detect C1q-adiponectin complexes in the serum by using an anti-human adiponectin F(ab')2 antibody as the plate-immobilized antibody, and using an anti-human C1q antibody as the detection antibody.

The presence form of adiponectin complexes in human serum and its clinical significance were clarified for the first time in the world by using the C1q-adiponectin complex ELISA method established in the present invention.

First, the present inventors subjected human serum to molecular weight fractionation by gel filtration chromatography, and measured the abundance of C1q-adiponectin complexes in each fraction. This analysis revealed that C1q-adiponectin complexes were present in the same molecular weight size as that of adiponectin in high-molecular-weight and medium-molecular-weight fractions.

Next, for the purpose of evaluating the clinical significance of C1q-adiponectin complexes, the present inventors measured blood levels of C1q-adiponectin complexes of 329 healthy subjects. In this analysis, the ratio of the abundance of C1q-adiponectin complexes (C1q-APN) to the total amount of adiponectin (Total-APN) (C1q-APN/Total-APN ratio) was developed as a new blood indicator. Interestingly, this indicator showed significantly higher values in MetS associated with visceral fat accumulation and a high-risk group of cardiovascular disease, compared to a non-risk group.

The above results indicated that the present measurement method and analysis method allowed evaluation from a new angle, i.e., qualitative changes of adiponectin, in addition to quantitative changes of adiponectin in the blood. Therefore, the clinical use of the present indicator is very important to efficiently extract MetS associated with visceral fat accumulation or a high-risk group of arteriosclerotic disease.

That is, the present invention includes the following embodiments.

Item 1. A C1q-adiponectin complex comprising C1q and naturally occurring adiponectin.
Item 2. A method for measuring the amount of the C1q-adiponectin complex according to Item 1, the method comprising measuring the amount of the complex by an immunoassay.
Item 3. The measurement method according to Item 2, wherein the immunoassay is an ELISA method.
Item 4. The measurement method according to Item 3, wherein the ELISA method is a sandwich ELISA method using an anti-adiponectin antibody and an anti-C1q antibody.
Item 5. The measurement method according to Item 4, wherein the anti-adiponectin antibody is F(ab')2.
Item 6. A method for diagnosing metabolic syndrome or arteriosclerosis risk, the method comprising measuring the amount of the C1q-adiponectin complex according to Item 1 in blood taken from a diagnosis target, by the measurement method according to any one of Items 2 to 5.
Item 7. The diagnosis method according to Item 6, which uses, as an indicator, a ratio of the amount of the C1q-adiponectin complex according to Item 1 to the total adiponectin amount (C1q-APN/Total-APN ratio) in the blood.
Item 8. A kit for measuring the amount of the C1q-adiponectin complex according to Item 1, the kit comprising an anti-adiponectin antibody and an anti-C1q antibody.
Item 9. The kit according to Item 8, which is for diagnosis of metabolic syndrome or arteriosclerosis risk.

### Advantageous Effects of Invention

The present invention provides a C1q-adiponectin complex that is useful as a biomarker for metabolic syndrome or arteriosclerosis, and a measurement method thereof. Moreover, the present invention allows for a more precise diagnosis of MetS or arteriosclerosis risk than the conventional total adiponectin measurement method and high-molecular-weight adiponectin measurement method.

### Brief Description of Drawings

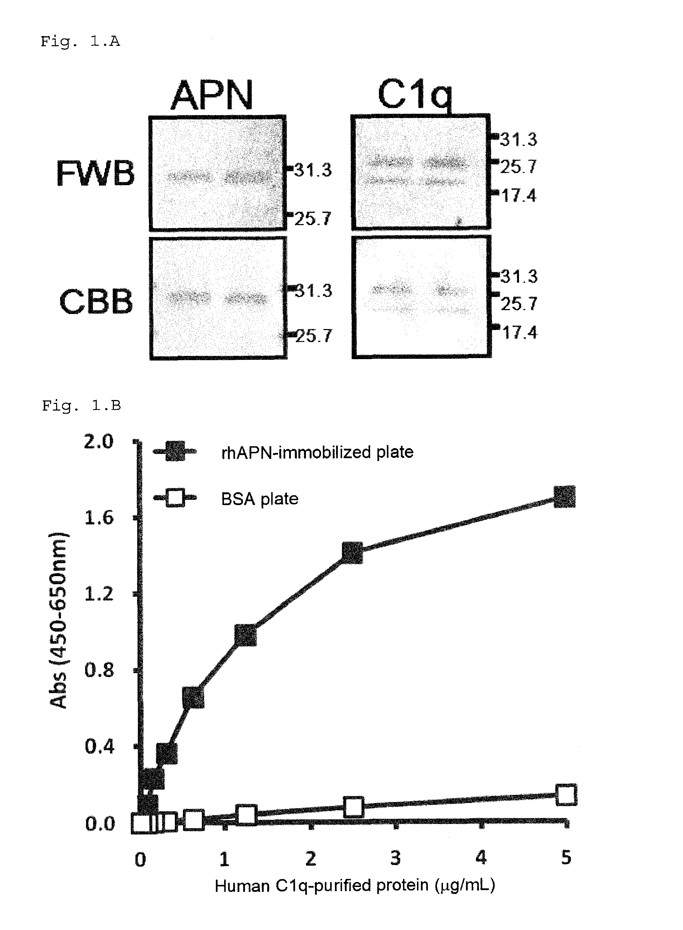

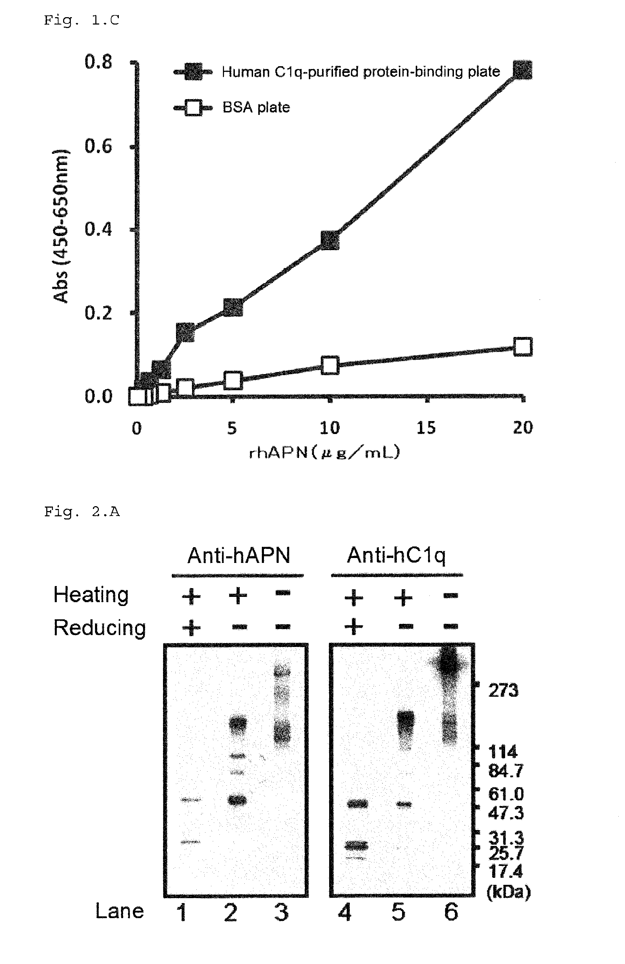

Fig. 1.A shows photographs of far-Western blotting in binding analysis of human adiponectin recombinant protein and complement C1q-purified protein (Example 1).
Fig. 1.B is a graph showing the binding evaluation results of binding analysis of human adiponectin recombinant protein and complement C1q-purified protein (Example 1).
Fig. 1.C is a graph showing the binding evaluation results of binding analysis of human adiponectin recombinant protein and complement C1q-purified protein (Example 1).
Fig. 2.A shows photographs of Western blotting indicating the detection of multimeric structures of adiponectin and C1q in binding analysis of adiponectin and C1q in human serum (Example 2).

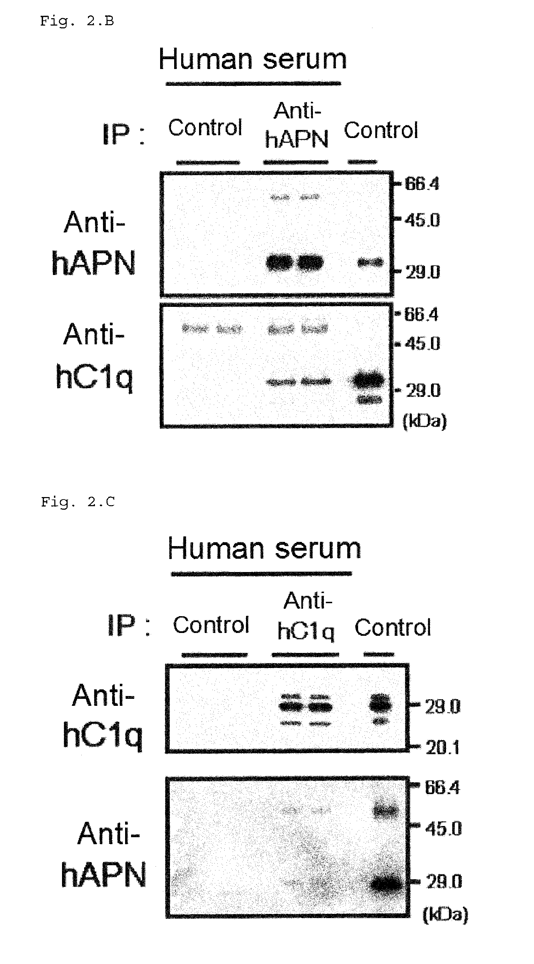

Fig. 2.B shows photographs of Western blotting indicating the results of adiponectin immunoprecipitation in binding analysis of adiponectin and C1q in human serum (Example 2).
Fig. 2.C shows photographs of Western blotting indicating the results of C1q immunoprecipitation in binding analysis of adiponectin and C1q in human serum (Example 2).

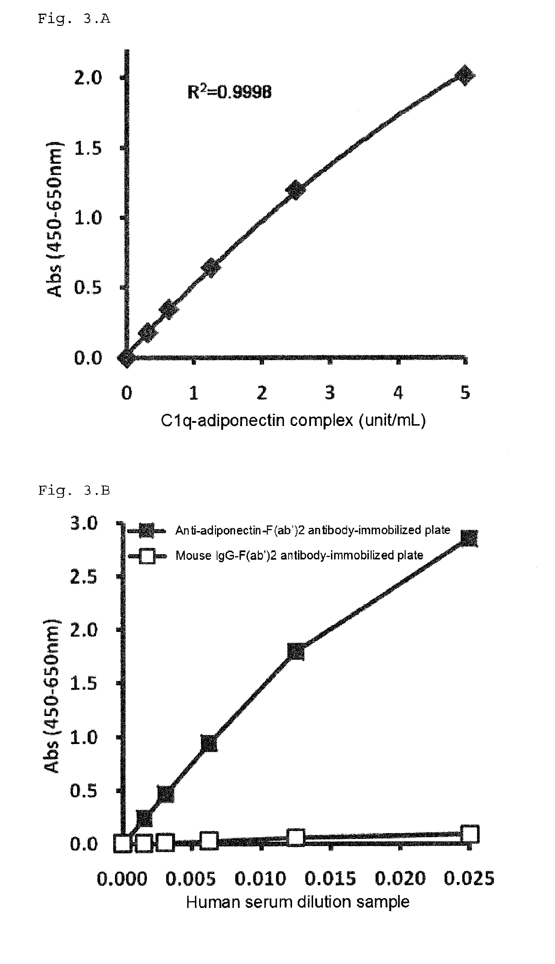

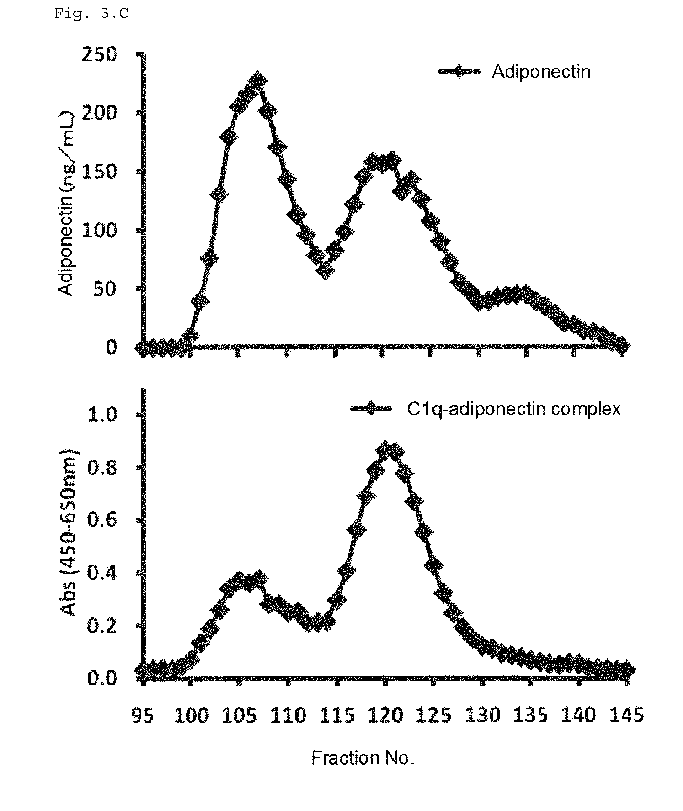

Fig. 3.A shows a standard curve prepared in the development of an ELISA method (Examples 3 and 4).
Fig. 3.B shows a dilution curve prepared in the development of an ELISA method (Examples 3 and 4).
Fig. 3.C shows graphs indicating the form of gel filtration fractionation of C1q-adiponectin complexes in human serum (Examples 3 and 4).

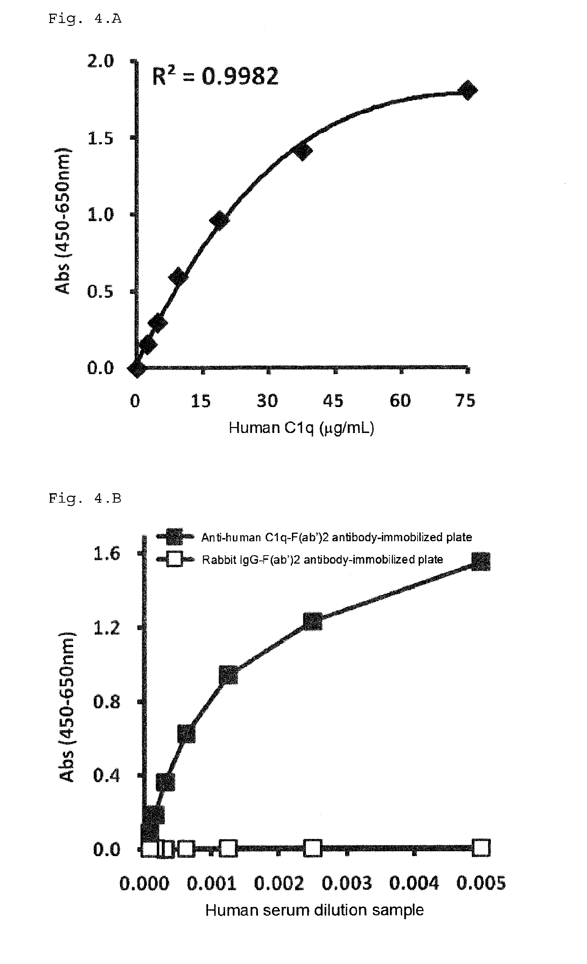

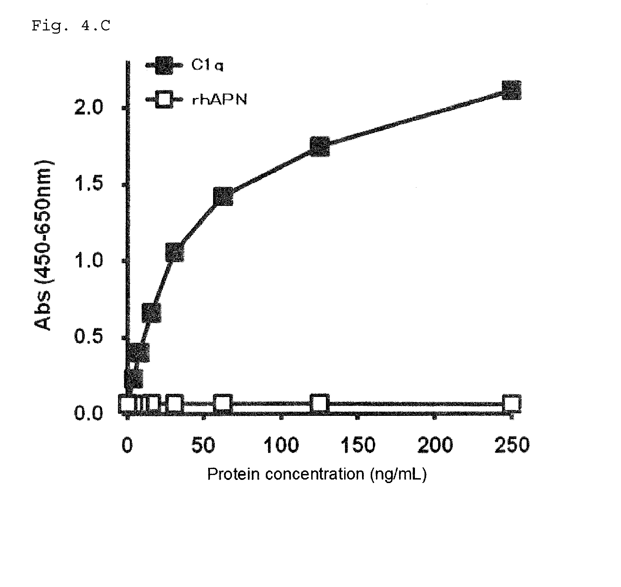

Fig. 4.A shows a standard curve prepared in the establishment of an ELISA method for measuring C1q in human serum (Example 5).
Fig. 4.B shows a graph indicating the results of measuring the amount of C1q in human serum (Example 5).
Fig. 4.C shows a graph indicating the correlation between protein concentration and absorbance (Example 5).

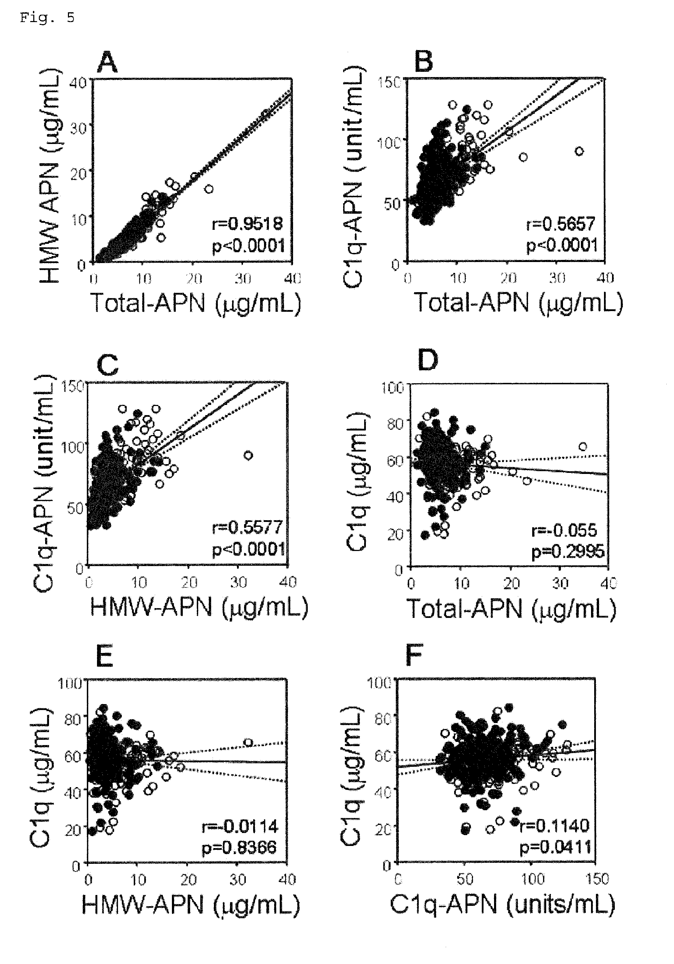

Fig. 5 shows graphs indicating the correlation between Total-APN, HMW-APN, C1q-APN, and C1q in the serum of 329 healthy subjects (Example 6).

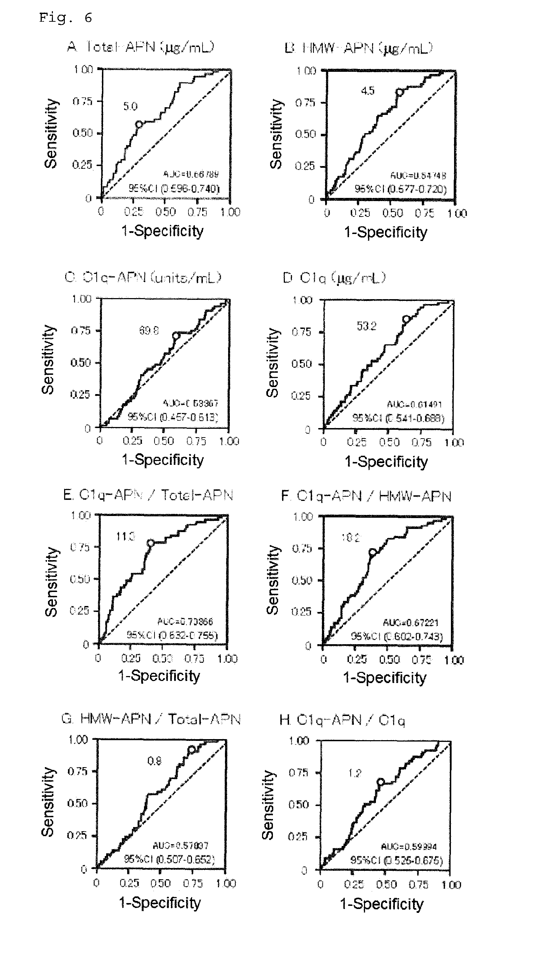

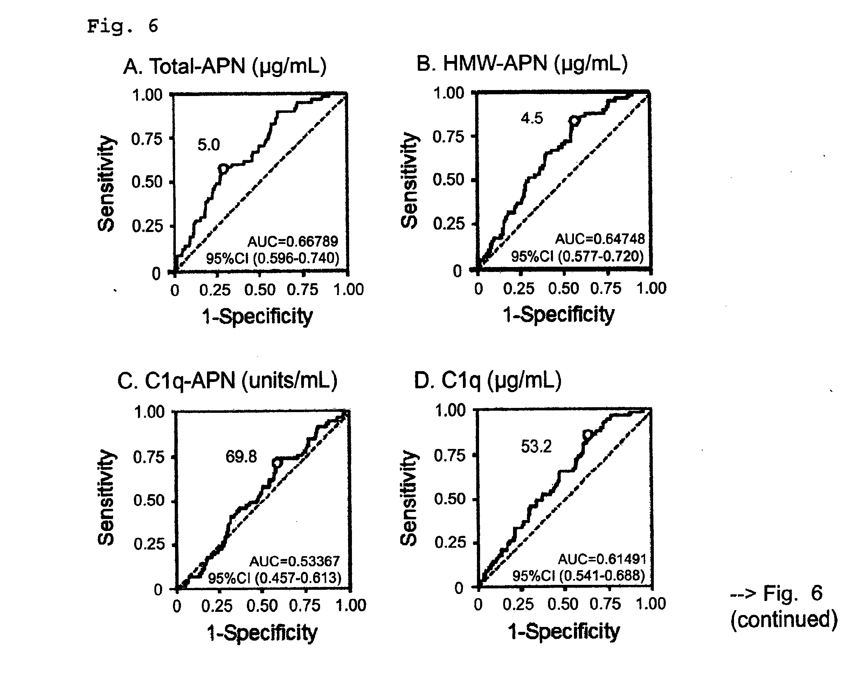

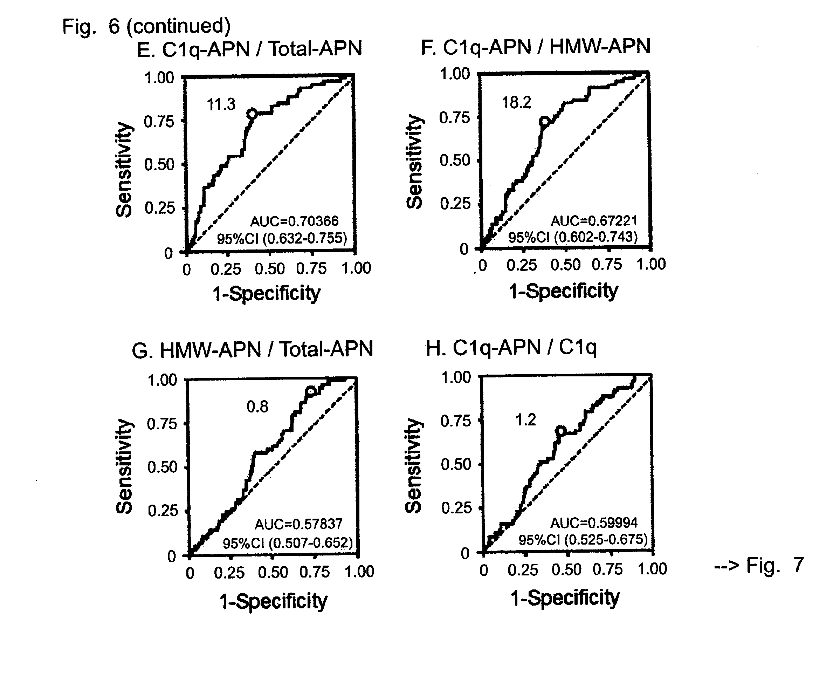

Fig. 6 shows graphs indicating ROC (Receiver Operating Characteristic) curve analysis for the determination of the presence of metabolic syndrome in 329 healthy subjects (Example 7).

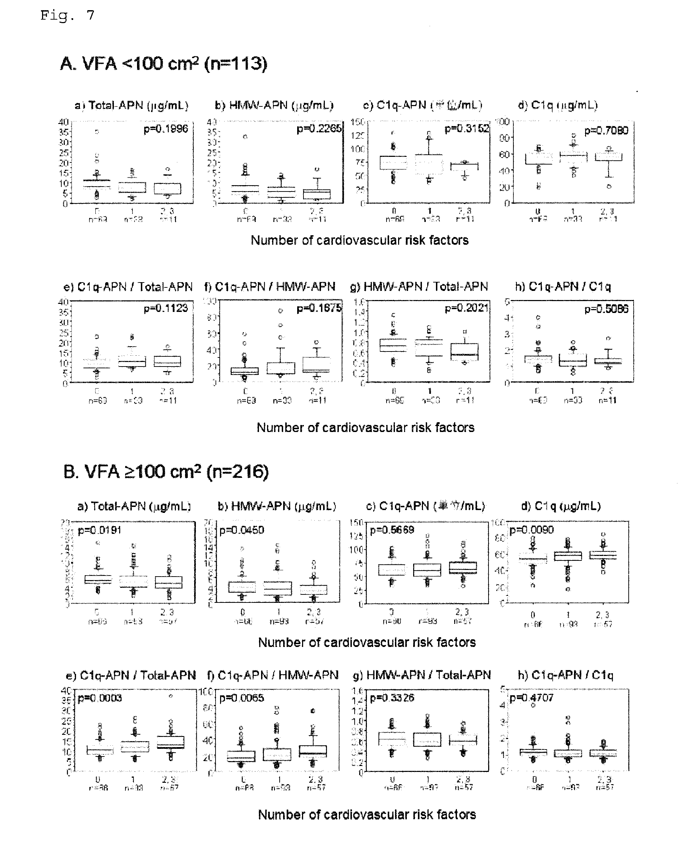

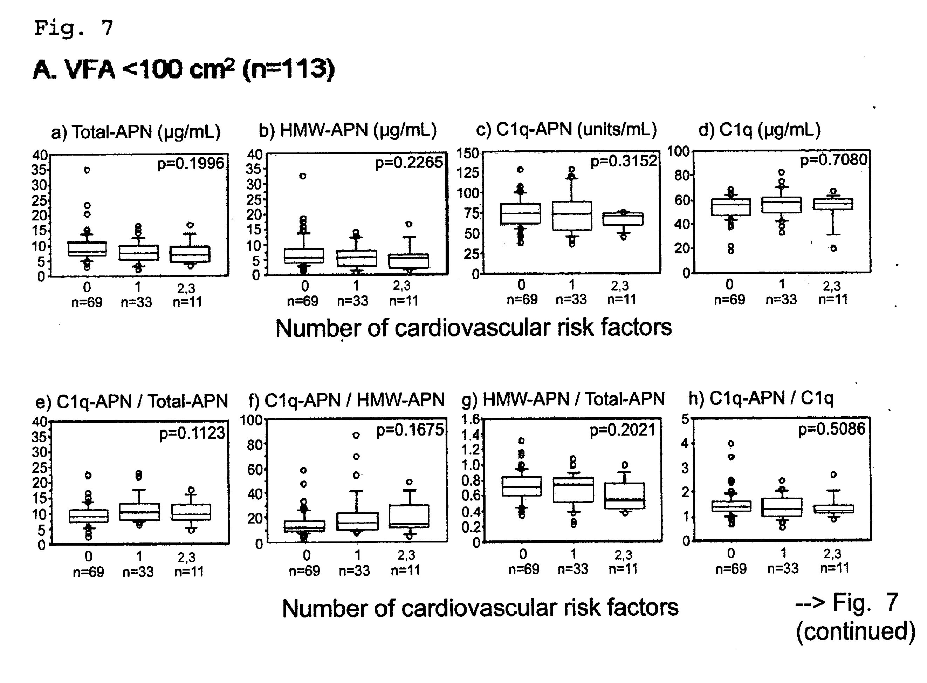

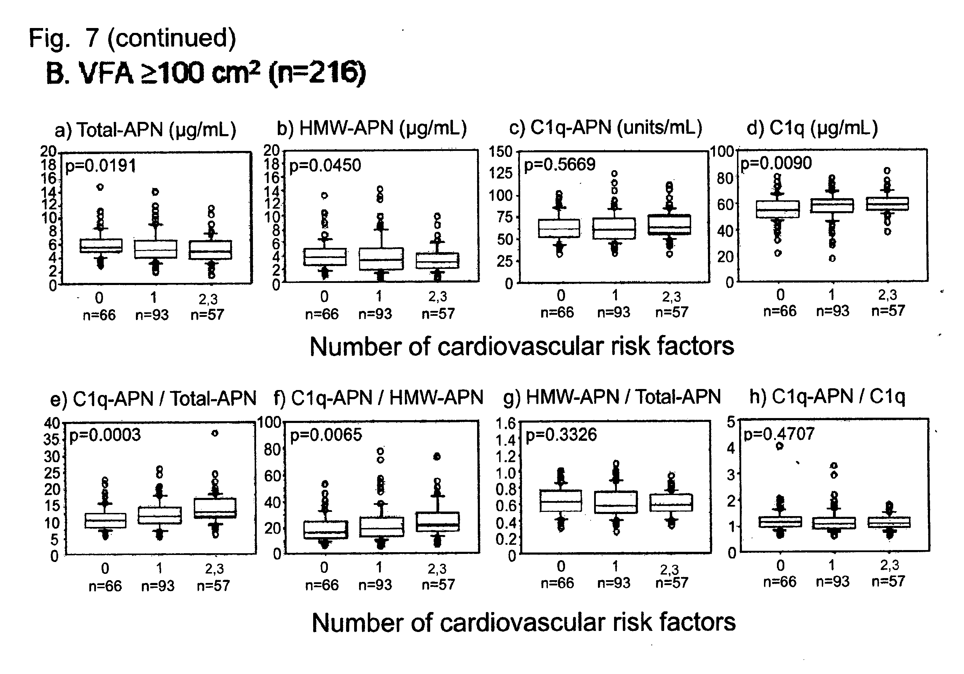

Fig. 7 shows graphs indicating the relation between various adiponectin-related indicators in visceral fat accumulation and obesity-related cardiovascular risk factor accumulation (box-and-whisker plot) (Example 8).

### Description of Embodiments

### 1. C1q-Adiponectin Complex

The C1q-adiponectin complex of the present invention comprises naturally occurring adiponectin and C1q. The C1q-adiponectin complex of the present invention preferably consists of naturally occurring adiponectin and C1q.

### 1.1. Naturally Occurring Adiponectin

Adiponectin is a bioactive protein comprising 244 amino acids, and is secreted from adipose cells. Adiponectin has a signal peptide in the N terminus, collagen-like domain, and a spherical domain in the C terminus. In particular, the spherical domain is known to possess high homology to collagen V or VIII, and complement factor C1q (NPL 17). In humans, adiponectin reportedly is present at a concentration of 4 to 30 µg/mL in blood, which decreases with the accumulation of visceral fat (NPL 18). Since adiponectin has a characteristic three-dimensional structure, including a collagen-like domain, etc., various three-dimensional structures, such as trimers, hexamers, and higher-molecular-weight (HMW) multimers, are presumably formed in the blood (NPL 19).

The naturally occurring adiponectin in the present invention may be any of these forms, or a mixture thereof.

NPL 17: Okamoto Y, Arita Y, Nishida M, et al., An adipocyte-derived plasma protein, adiponectin, adheres to injured vascular walls, Horm Metab Res, 2000; 32: 47-50
NPL 18: Arita Y, Kihara S, Ouchi N, et al., Paradoxical decrease of an adipose-specific protein, adiponectin, in obesity, Biochem Biophys Res Commun, 1999; 257: 79-83
NPL 19: Pajvani UB, Du X, Combs TP, Berg AH, Rajala MW, Schulthess T, Engel J, Brownlee M, Scherer PE, Structure-function studies of the adipocyte-secreted hormone Acrp30/adiponectin, Implications for metabolic regulation and bioactivity, J Biol Chem, 2003 Mar 14; 278(11): 9073-85

In the present specification, the naturally occurring adiponectin refers to adiponectin isolated from nature. Accordingly, adiponectin having a structure modified by chemical treatment with an oxidant, etc., is excluded from the naturally occurring adiponectin in the present invention.

Adiponectin is naturally produced by adipose cells, and contained in blood, etc.

The naturally occurring adiponectin is preferably adiponectin isolated from blood. Note that the phrase "isolated from blood" as used in the present specification includes indirect isolation from blood. That is, for example, adiponectin isolated from serum is adiponectin isolated from blood.

The naturally occurring adiponectin is obtained by purifying roughly purified naturally occurring adiponectin extracted from blood by a known protein purification method, such as gel electrophoresis, dialysis, or affinity chromatography, as desired.

On the other hand, the naturally occurring adiponectin in the present invention also includes adiponectin having the same structure as that of adiponectin isolated from nature.

The naturally occurring adiponectin may be recombinant adiponectin produced by gene engineering techniques, as long as it has the same structure as that of adiponectin isolated from nature. Recombinant adiponectin can be produced by genetic recombination of E. coli, yeast, or mammalian cell lines (e.g., 293 cells, CHO cells) according to well-known conventional techniques in this field.

### 1.2. C1q

C1q is a protein of approximately 462 kDa, composed of 18 subunits (6 molecules of A chain, 6 molecules of B chain, and 6 molecules of C chain). According to NPL 20, C1q is present at 80 µg/mL in human blood. C1q is a main subcomponent of the first component C1 of the complement activation pathway, and recognizes antigen-antibody complexes.

C1q in the present invention is not particularly limited. For example, C1q isolated from nature can be used.

### NPL 20: Sunyer, J.O. and Lambris, J.D. (1999), Complement, Encyclopedia of Life Sciences

C1q can be prepared by a known method (e.g., the method disclosed in JPH09-178745A) in which a C1q roughly purified product solution obtained by dialysis of serum or plasma is brought into contact with an IgG-immobilized insoluble carrier, and then C1q adhering to the insoluble carrier is separated and collected.

In the C1q-adiponectin complex of the present invention, naturally occurring adiponectin and C1q are bonded directly and non-covalently.

The weight ratio of naturally occurring adiponectin and C1q in the C1q-adiponectin complex of the present invention is generally 1:10 to 10:1, preferably 1:3 to 3:1, and more preferably about 1:1.

The C1q-adiponectin complex of the present invention is obtained by mixing naturally occurring adiponectin and C1q at the above ratio, and stirring the mixture in an aqueous solvent (e.g., a phosphate buffer).

The stirring time is generally 1 to 48 hours, and preferably 12 to 36 hours.

The stirring temperature is preferably 20 to 40°C.

### 2. Method for Measuring the Amount of C1Q-Adiponectin Complexes

The method of the present invention for measuring the amount of C1q-adiponectin complexes (hereinafter also referred to as simply "the measurement method of the present invention") characteristically comprises measuring the amount of the complex by immunoassay.

The biological samples that can be used in the measurement method of the present invention are not particularly limited as long as they contain C1q-adiponectin complexes. Examples thereof include body fluids such as blood (serum, plasma), tissue extract fluids, culture supernatant fluids of tissue-derived cells and the like, which are obtained from humans, monkeys, goats, sheep, rabbits, mice, rats, guinea pigs, or other mammalian animals. When the purpose of measurement is to diagnose MetS or arteriosclerosis risk in humans, a preferred biological sample is human blood (serum, plasma).

The method for obtaining samples may be suitably determined depending on the purpose of measurement, etc. For example, samples are obtained by collecting blood from a subject in a fasting state.

Usable examples of immunoassays include Western blotting, dot blotting, immunoprecipitation, enzyme immunoassay (EIA), enzyme-linked immunosorbent assay (ELISA), radioimmunoassay (RIA), fluorescent immunoassay (FIA), immunohistostaining, immunochromatography, and other known immunoassays.

These immunoassays may be performed according to conventional methods.

The immunoassay in the measurement method of the present invention uses an antibody that recognizes C1q-adiponectin complexes. The antibody can be used without limitation as long as it can recognize C1q-adiponectin complexes. The antibody may be a specific antibody against C1q-adiponectin complexes (anti-C1q-adiponectin complex antibody) or a combination of anti-adiponectin antibody and anti-C1q antibody. A combination of anti-adiponectin antibody and anti-C1q antibody is preferred.

These antibodies may be monoclonal or polyclonal antibodies.

These antibodies can be obtained, for example, by using adiponectin, C1q, or a partial peptide thereof as an antigen. These antibodies can also be obtained as commercial products.

The following explains the method for producing the anti-adiponectin antibody and anti-C1q antibody.

Monoclonal antibodies can be produced by a known method. Specifically, a warm-blooded animal (e.g., monkey, rabbit, dog, guinea pig, mouse, rat, sheep, goat, or chicken) is sensitized with an antigen, hybridomas of immune-related cells of the immunized animal and myeloma cells are produced, and hybridomas that produce the target specific antibody are selected from the produced hybridomas.

As adiponectin and C1q used as antigens to sensitize a warm-blooded animal, the above-explained adiponectin and C1q, or partial peptides thereof can be used. The partial peptides can be obtained by a known production method, such as cutting adiponectin and C1q with suitable peptidases, or synthesis by a chemical synthesis method (e.g., immobilization synthesis).

Such an antigen may be administered to an aforementioned animal directly or in the form of a mixture or emulsion with an adjuvant, such as Freund's complete adjuvant or Freund's incomplete adjuvant, in order to enhance the antibody production ability of the immunized animal.

Generally, immunization is carried out using 0.1 to 100 µg/body of antigen once to several times (approximately every other week). Individuals confirmed to have antibody titer are selected, and the spleen, thymus, or lymph gland is taken within two to five days after the final immunization. Cells obtained from these organs are fused with a mouse myeloma cell line (e.g., SP2/0 or P3U1) by a known method, such as the polyethylene glycol method, thereby producing hybridomas.

The screening of hybridomas can be performed by a known method. For example, a hybridoma culture supernatant is added to a solid phase in which the antigen is adsorbed, and then reacted with a labeled anti-immunoglobulin antibody, etc., thereby detecting the monoclonal antibody binding to the solid phase.

Polyclonal antibodies can be produced by immunizing a warm-blooded animal, taking a substance containing antibodies against the proteins of the present invention from the immunized animal, and isolating and purifying the antibodies, as in the above-described method for producing monoclonal antibodies.

An ELISA method is preferred as the immunoassay in the measurement method of the present invention. A sandwich method is also preferred as the immunoassay in the measurement method of the present invention. A sandwich ELISA method is particularly preferred as the immunoassay in the measurement method of the present invention. The sandwich ELISA method is carried out by a known method under known conditions.

A preferred embodiment of the sandwich ELISA in the measurement method of the present invention comprises the steps of:
insolubilizing an anti-adiponectin antibody;
bringing a sample into contact with the insolubilized antibody to bind C1q-adiponectin complexes in the sample to the antibodies;
binding an anti-C1q antibody to the C1q-adiponectin complexes; and
binding a labeled antibody (secondary antibody) to the anti-C1q antibody binding to the C1q-adiponectin complexes.

The anti-adiponectin antibody can be insolubilized, for example, by chemically or physically binding the anti-adiponectin antibody to a plate or another container wall, or a known carrier, such as beads, slides, resin films, or columns.

The antibodies used in the measurement method may be complete antibody molecules; fragments, such as Fab, Fab', F(ab')2, Fv, and VH; conjugated molecules produced by gene engineering, such as scFv, scFv-Fc, dsFv, minibodies, and diabodies; or derivatives modified with molecules, etc., having a protein stabilizing effect, such as polyethylene glycol (PEG).

Since C1q may undesirably bind to the Fc region of the anti-adiponectin antibody, the anti-adiponectin antibody is preferably F(ab')2.

The above fragments can be obtained by treating the antibodies with an enzyme, such as papain or pepsin, or by constructing genes encoding these antibody fragments, and allowing the genes to express in any host cells.

The labeled antibody (secondary antibody) to be bonded to the anti-C1q antibody is, for example, an IgG antibody labeled by a known method with an enzyme, such as horseradish peroxidase (HRP), alkaline phosphatase, or β-galactosidase; a radioactive isotope, such as ¹²⁵I, ³²P, ¹⁴C, ³⁵S, or ³H; a fluorescent compound, such as fluorescein (FITC) or tetramethyl rhodamine isocyanate; or the like.

### 3. Method for Diagnosing Metabolic Syndrome or Arteriosclerosis Risk

The method of the present invention for diagnosing metabolic syndrome or arteriosclerosis risk comprises measuring the amount of C1q-adiponectin complexes in the blood taken from a diagnosis target.

In the method of the present invention for diagnosing metabolic syndrome or arteriosclerosis risk, the amount of C1q-adiponectin complexes or numerical values derived from the amount are used as an indicator to diagnose metabolic syndrome or arteriosclerosis risk.

That is, the C1q-adiponectin complex is effectively used as a marker for metabolic syndrome or arteriosclerosis.

Examples of the numerical values used as the indicator in the diagnosis method of the present invention include:
the amount of C1q-adiponectin complexes in the blood;
the ratio of the C1q-adiponectin complex amount to the total adiponectin amount (Clq-APN/Total-APN ratio) in the blood; and
the ratio of the C1q-adiponectin complex amount to the high-molecular-weight adiponectin (HMW-APN) amount (C1q-APN/HMW-APN ratio) in the blood.

Preferred among these in terms of high correlation with metabolic syndrome or arteriosclerosis are the C1q-APN/Total-APN ratio or C1q-APN/HMW-APN ratio, and more preferred is the C1q-APN/Total-APN ratio. It is known that the high-molecular-weight adiponectin amount and the total adiponectin amount are highly correlated with each other.

The total adiponectin amount and the high-molecular-weight adiponectin amount used in the calculation of these numerical values can be measured by known methods. Examples of known methods include the high-molecular-weight adiponectin measurement method disclosed in PTL 1, mentioned above. Alternatively, the measurement may be performed using a commercially available measurement kit.

In the diagnosis of metabolic syndrome or arteriosclerosis risk, numerical values obtained from a diagnosis target are compared, for example, with reference values (or cutoff points) determined on the basis of numerical values obtained from subjects who are known to have not suffered from metabolic syndrome or arteriosclerosis, and numerical values obtained from subjects who are known to have suffered from metabolic syndrome or arteriosclerosis.

For example, when a Clq-APN/Total-APN ratio is used, a subject has a C1q-APN/Total-APN ratio higher than its reference value is diagnosed with metabolic syndrome or arteriosclerosis, or with a high risk of metabolic syndrome or arteriosclerosis.

These indicators may be used singly; or in diagnosis of metabolic syndrome or arteriosclerosis risk, these indicators may be used in combination with other indicators used in the diagnosis thereof.

### 4. Kit

The present invention also provides a kit for selectively measuring C1q-adiponectin complexes using the above method.

The kit of the present invention comprises an anti-C1q-adiponectin complex antibody or a combination of an anti-adiponectin antibody and an anti-C1q antibody.

The above-mentioned examples of these antibodies can be used.

The anti-C1q-adiponectin complex antibody, or either one of the anti-adiponectin antibody and the anti-C1q antibody preferably binds to an ELISA plate.

The kit preferably comprises an anti-C1q-adiponectin complex antibody as standard.

The kit preferably comprises instructions for use.

### Examples

Examples of the present invention are shown below; however, the present invention is not limited thereto.

First, the materials and methods of the Examples of the present invention are described.

### 1-1. Far-Western Blotting Method

### (1) Evaluation of Binding of Adiponectin to C1q

Biotin labeling of recombinant adiponectin protein (rhAPN, 200 µg) was performed using EZ-Link Sulfo-NHS-LC-biotin (Pierce) according to the package insert. Human C1q-purified protein (100 ng) was separated by SDS-polyacrylamide gel electrophoresis (SDS-PAGE), and then transferred to a nitrocellulose membrane (Protran BA 85 Nitrocellulose Membrane; Whatman). The membrane was blocked with Block Ace (DS Pharma) at room temperature for 1 hour. Then, the membrane was incubated with biotin-labeled rhAPN diluted to 1 µg/mL at 4°C for 18 hours. After washing three times with PBST, the membrane was stirred with 10,000-fold diluted HRP-labeled streptavidin (Invitrogen) at room temperature for 1 hour. Finally, after washing three times again, detection was performed using ECL Plus Western Blotting Detection Reagent (GE Healthcare).

### (2) Evaluation of Binding of C1q to Adiponectin

rhAPN (200 ng) was separated by SDS-PAGE, and then transferred to a nitrocellulose membrane. The membrane was blocked with Block Ace (DS Pharma) at room temperature for 1 hour. Then, the membrane was incubated with human C1q-purified protein diluted to 1 µg/mL at room temperature for 3 hours. After washing three times with PBST, the membrane was stirred with 1,000-fold diluted HRP-labeled anti-human C1q polyclonal antibody at room temperature for 1 hour. Finally, after washing three times again, detection was performed using ECL Plus reagent, as in evaluation (1) above.

### 1-2. Immunoprecipitation

An anti-human adiponectin F(ab')2 antibody and anti-human C1q-F(ab')2 antibody (each 100 µg) were coupled to magnetic beads (Dynabeads M-280 Tosylactivated; Invitrogen) according to the package insert. Human serum diluted with a diluent for sample (PBS(-) containing 1% Tween20, 1 M NaCl, and 0.1% ProClin 300) was mixed with the above antibody-binding magnetic beads, and mixed by inversion at 4°C for 18 hours. After the magnetic beads were washed three times with a sample diluent, the immunoprecipitated products were each used in SDS-PAGE and Western blotting.

### 1-3. Human C1Q-Adiponectin Complex ELISA method

As the anti-adiponectin monoclonal antibody, an F(ab')2 antibody was produced using a commercially available kit (ImmunoPure IgG1 Fab and F(ab')2 Preparation Kit, Pierce) according to the instructions for use. The anti-adiponectin F(ab')2 antibody (100 µL) adjusted to 3 µg/mL was added to a 96-well microtiter plate for EIA/RIA (96-Well EIA/RIA 1x8 Stripwell Plate, Corning), and immobilized by incubation at 4°C for 18 hours. Then, the plate was washed once with a washing solution (D-PBS(-) containing 0.05% Tween20), and 300 µL of blocking solution (D-PBS(-) containing 1% bovine serum albumin (BSA) and 5% D-sorbitol) was added, followed by incubation at 4°C for 18 hours. After removing the blocking solution, the plate was dried in a dry room and stored at 4°C before use.

A C1q-adiponectin (APN) complex reference standard was produced by mixing 10 µg/mL of recombinant human adiponectin protein and 10 µg/mL of human C1q-purified protein, and stirring the mixture at 37°C for 24 hours. An 800-fold dilution of the reference standard was defined as an arbitrary amount of C1q-APN complexes at 1 unit/mL.

For standard curve generation, the C1q-APN complex reference standard produced above was diluted with a diluent for sample (PBS(-) containing 1% BSA, 1% pig serum, 0.1% Tween20, 1 M NaCl, and 0.1% ProClin 300), thereby preparing diluted solutions of 5, 2.5, 1.25, 0.625, 0.313, and 0.156 unit/mL. Each standard solution (100 µL) and 100 µL of human serum (80-fold diluted with a sample diluent) were added to each well, and incubated at 25°C for 1 hour. Next, after each well was washed three times with 350 µL of washing solution, 100 µL of anti-human C1q polyclonal antibody (DAKO), which had been 5,000-fold diluted with a diluent for primary antibody (PBS(-) containing 1% BSA, 1% pig serum, 0.1% Tween20, 1 M NaCl, and 0.1% ProClin 300), was added and incubated at 25°C for 1 hour. Then, after washing three times, 100 µL of horseradish peroxidase (HRP)-labeled anti-rabbit IgG antibody, which had been 10,000-fold diluted with a diluent for secondary antibody (PBS(-) containing 1% BSA, 1% pig serum, 0.1% Tween20, and 0.1% ProClin 300), was added and incubated at 25°C for 1 hour. Finally, after each well was washed three times with a washing solution, 100 µL of 3,3',5,5'-tetramethylbenzidine (TMB) coloring solution was added to perform color development at room temperature for 15 minutes. To the wells after color development, 100 µL of 1 N dilute sulfuric acid was added to terminate the reaction, and the absorbance at 450 nm (reference wave length: 650 nm) (Abs (450 to 650 nm)) was measured by a Vmax microplate reader (produced by Molecular Devices). The amount of C1q-APN complexes in the serum was calculated by generating a standard curve using Softmax Pro software (produced by Molecular Devices).

### 1-4. Gel Filtration Fractionation of Human Serum

Human serum (150 µL) was filtered through a 0.22-µm syringe filter, and then fractionated by Superdex-200 (GE Healthcare) chromatography connected to an AKTAexplorer 10S (GE Healthcare). Each fraction was collected at a flow rate of 0.5 mL/min, while measuring the absorption at 280 nm.

### 1-5. Human C1q ELISA Method

An anti-human C1q-F(ab')2 antibody was produced by the pepsin digestion method. An anti-human C1q polyclonal antibody (500 µg) was diluted with a 0.1 M sodium acetate buffer at pH 4.4, and digested with 20 µg of pepsin (Wako Pure Chemical Industries) at 37°C for 18 hours. Thereafter, the F(ab')2 antibody was purified by Protein A chromatography and Sephacryl S-300 (GE Healthcare) chromatography. The anti-human C1q-F(ab')2 antibody (100 µL) adjusted to 3 µg/mL was added to a 96-well microtiter plate for EIA/RIA, and immobilized by incubation at 4°C for 18 hours. Then, the plate was washed once with a washing solution, and 300 µL of blocking solution was added, followed by incubation at 4°C for 18 hours. After removing the blocking solution, the plate was dried in a dry room and stored at 4°C before use.

As human C1q reference standards, human C1q-purified protein was diluted with a diluent for sample and antibody (PBS(-) containing 0.5% BSA, 0.05% Tween20, and 0.05% ProClin 300), thereby preparing diluted solutions of 150, 75, 37.5, 18.75, 9.38, 4.69, and 2.34 ng/mL. Each standard solution (100 µL) and 100 µL of human serum (3,000-fold diluted with a sample diluent) were added to each well, and incubated at 25°C for 1 hour. Next, after each well was washed three times with 350 µL of washing solution, 100 µL of HRP-labeled anti-human C1q polyclonal antibody, which had been 40,000-fold diluted with a diluent for sample and antibody, were added to each cell, and incubated at 25°C for 1 hour. Subsequently, detection and assay were performed in the same manner as in the above C1q-APN complex ELISA method. The HRP-labeled anti-human C1q polyclonal antibody used as the detection antibody in this method was produced using the Peroxidase Labeling Kit-NH₂ (DOJINDO) according to the package insert.

### Example 1: Evaluation of in vitro Binding between Adiponectin and C1q

Fig. 1 shows the results of the evaluation of binding between adiponectin and C1q by far-Western blotting. After SDS-PAGE of recombinant adiponectin protein (rhAPN) was performed under reducing and heating conditions, the blotted membrane was reacted with human C1q-purified protein, and detection was performed using an anti-human C1q polyclonal antibody (Fig. 1.A, left (APN)). Similarly, a membrane blotted with human C1q-purified protein was reacted with biotin-labeled rhAPN, and further reacted with HRP labeled-streptavidin (Fig. 1.A, right (Clq)). Direct binding between adiponectin and C1q was demonstrated *in vitro* in the above manner.

Next, binding was evaluated using an rhAPN-binding plate and a human C1q-purified protein-binding plate. Fig. 1.B shows the results obtained when human C1q-purified protein was added at the concentrations shown in the figure to a rhAPN-immobilized plate (1 µg/mL), and detection was performed using an anti-human C1q antibody. Further, Fig. 1.C shows the results obtained when rhAPN was added at the concentrations shown in the figure to a human C1q-purified protein-immobilized plate (1 µg/mL), and detection was performed using an anti-human adiponectin antibody. Direct binding between adiponectin and C1q was also confirmed from the binding experiments using these ELISA plates. In contrast, rhAPN and human C1q-purified protein both showed almost no binding to BSA plates (control).

### Example 2: Detection of C1q-Adiponectin Complex in Human Serum

Human serum was subjected to SDS-PAGE and Western blotting under reducing and heating conditions, non-reducing and heating conditions, or non-reducing and non-heating conditions, thereby detecting multimeric structures of adiponectin and C1q. Fig. 2.A shows the results. In the treatment under heating and reducing conditions, a signal derived from adiponectin was detected at around 31 kDa, and signals derived from C1q were detected at around 31, 30, and 26 kDa. Comparatively, in the treatment under non-reducing and non-heating conditions, high-molecular-weight forms of adiponectin and C1q were detected at 114 kDa or more.

Next, C1q-adiponectin complexes present in the blood were directly detected by co-immunoprecipitation using human serum. Adiponectin and C1q in the serum were immunoprecipitated, and each of their immunoprecipitated products was subjected to Western blotting using an anti-human adiponectin antibody or anti-human C1q antibody. Fig. 2.B shows the results of adiponectin immunoprecipitation, and Fig. 2.C shows the results of C1q immunoprecipitation. As a result, signals specific to C1q were detected in the adiponectin immunoprecipitated product. Similarly, signals specific to adiponectin were detected in the C1q immunoprecipitated product. Thus, the examination results by co-immunoprecipitation demonstrated that C1q-adiponectin complexes were present in the human serum.

Binding between adiponectin and C1q has been reported so far by *in-vitro* studies (NPL 8). However, the presence of C1q-adiponectin complexes in the human serum has been demonstrated for the first time by the present invention.

### Example 3: Establishment of ELISA Method for Measuring C1q-Adiponectin Complex in Human Serum

For the purpose of analyzing the clinical significance of C1q-adiponectin complexes present in human serum, an ELISA method for measuring the amount of C1q-adiponectin complexes in the serum was developed. A complex reference standard and 80-fold diluted human serum were added to each well of an anti-adiponectin-F(ab')2 antibody-immobilized plate. An anti-human C1q polyclonal antibody and an HRP-labeled anti-rabbit IgG antibody were used as the detection antibodies. A standard curve was generated using a C1q-adiponectin complex reference standard that had been gradually diluted from 5 to 0.315 units/mL (Fig. 3.A). A good dilution curve was obtained from the human serum dilution sample, as shown in Fig. 3.B. No absorbance was observed in a mouse IgG-F(ab')2 antibody-immobilized plate, which was used as control.

### Example 4: Form of Gel Filtration Fractionation of C1q-Adiponectin Complex in Human Serum

Adiponectin is reportedly present in the circulating blood in fractionation patterns of a high-molecular-weight (HMW) fraction, a medium-molecular-weight (MMW) fraction, and a low-molecular-weight (LMW) fraction (Komura N, Kihara S, Sonoda M, et al., Clinical significance of high-molecular weight form of adiponectin in male patients with coronary artery disease, Circ J, 2008 Jan; 72(1): 23-8). In the present invention, the distribution form of C1q-adiponectin complexes present in human circulating blood was analyzed by gel-filtration chromatography. Human serum was fractionated by Superdex-200 chromatography, and the total adiponectin amount and the C1q-adiponectin complex amount in each fraction were measured. As shown in Fig. 3.C, it was suggested that C1q-adiponectin complexes were possibly present in adiponectin of the HMW and MMW fractions.

### Example 5: Establishment of ELISA Method for Measuring C1q in Human Serum

An ELISA method specific to serum C1q was established. A human C1q reference standard and 3,000-fold diluted human serum were added to each well of an anti-human C1q-F(ab')2 antibody-immobilized plate. An HRP-labeled anti-human C1q polyclonal antibody was used as the detection antibody. A standard curve was generated using a human C1q reference standard that had been gradually diluted from 150 to 2.34 ng/mL (Fig. 4.A). A good dilution curve was obtained from the diluted human serum, as shown in Fig. 4.B. No absorbance was observed in a rabbit IgG-F(ab')2 antibody-immobilized plate, which was used as control.

The subunit (C chain) of C1q and adiponectin reportedly have particularly high homology (Maeda K, Okubo K, Shimomura I et al., cDNA cloning and expression of a novel adipose specific collagen-like factor, apM1 (AdiPose Most abundant Gene transcript 1), Biochem Biophys Res Commun, 1996, Apr 16; 221(2): 286-9). Next, regarding the ELISA method for measuring human C1q established in the present invention, cross-reactivity with adiponectin was examined by measuring recombinant adiponectin protein (rhAPN). As shown in Fig. 4.C, no absorbance was observed when rhAPN was added at up to 250 ng/mL. It was concluded from the above examination that this ELISA method was highly specific to C1q in human serum.

### Example 6: Correlation between C1q-Adiponectin Complex Amount and C1q Amount in Circulating Blood

Serum was kindly provided from people who had undergone medical examination at the Hitachi Health Care Center in 2010 and agreed to cooperate on the present study after receiving an explanation of the study contents. The subjects were 329 males 30 to 74 years of age who had undergone an abdominal CT scan and had no history of serious disease (cancer, cerebrovascular disease, myocardial infarction). Table 1 shows the clinical background of each subject. The subjects were classified into two groups: a non-visceral fat obesity group with a visceral fat area (VFA) < 100 cm² (113 subjects, average VFA: 66 ± 24 cm²), and a visceral fat obesity group with VFA ≥ 100 cm² (216 subjects, average VFA: 149 ± 38 cm²).

The data in Table 1 is the average value ± SD (range) or the number of analysis objects (n).

The abbreviations are as follows:
BMI: body mass index
WC: waist circumference
VFA: visceral fat area
SFA: subcutaneous fat area
SBP: systolic blood pressure
DBP: diastolic blood pressure
BG: blood glucose
fIRI: fasting insulin resistance index
TC: total cholesterol
HDL-C: high-density lipoprotein cholesterol
eGFR: estimated glomerular filtration rate (= 194 x
Cr - 1.094 x age - 0.287)
AST: aspartate aminotransferase
ALT: alanine aminotransferase
γ-GTP: γ-glutamyl transferase
Total-APN: total adiponectin
HMW-APN: high-molecular-weight adiponectin
C1q-APN: C1q-adiponectin complex

**Table 1**

| | Total (n=329) | VFA <100 cm² (n=113) | VFA ≥100 cm² (n=216) |
|---|---|---|---|
| Age | 52±10 | 50±11 | 53±9 |
| BMI, kg/m² | 23.9±2.8 | 22.0±2.0 | 25.0±2.7 |
| WC, cm | 84±8 | 78±6 | 87±7 |
| VFA, cm² | 121±52 | 66 ± 24 | 149 ± 38 |
| SFA, cm² | 132±54 | 99±42 | 149±52 |
| SBP, mmHg | 121±78 | 117±11 | 123±11 |
| DBP, mmHg | 78±8 | 75±8 | 79±7 |
| BG, mg/dL | 106±12 | 103±11 | 108±12 |
| fIRI, mIU/L | 6.4±3.9 | 4.3±1.7 | 7.4±4.3 |
| HbA1c (NGSP), % | 5.7±0.4 | 5.6±0.4 | 5.7±0.4 |
| TC, mg/dL | 206±32 | 199±29 | 210±32 |
| Triglyceride, mg/dL | 142±99 | 105±65 | 162±109 |
| HDL-C, mg/dL | 56±12 | 60±12 | 53 ± 12 |
| Creatinine, mg/dL | 0.9±0.1 | 0.9±0.1 | 0.9±0.1 |
| Uric acid, mg/dL | 6.1±1.2 | 5.8±1.2 | 6.3±1.1 |
| eGFR, ml/min/1.73m² | 74.8±12.6 | 77.1±12.2 | 73.5±12.6 |
| AST, IU/L | 24±9 | 22±8 | 25±10 |
| ALT, IU/L | 28±19 | 21±12 | 31±21 |
| γ-GTP, IU/L | 49±45 | 36±33 | 55±49 |
| Serum C1q, µg/mL | 56.0±10.0 | 54.2±10.2 | 57.0±9.8 |
| Serum Total-APN, µg/mL | 6.7±3.5 | 8.6±4.5 | 5.7±2.3 |
| Serum HMW-APN, µg/mL | 4.7±3.6 | 6.4±4.6 | 3.8±2.4 |
| Serum C1q-APN, unit/ml | 66.8±18.2 | 73.8±19.8 | 63.2±16.1 |
| Smoking (no/past/present) | n=85/149/95 | | |
| Brinkman index | 370 ± 348 | 324 ± 317 | 394 ± 362 |
| Hypertension (%) | 23.4 | 14.2 | 28.2 |
| Abnormal glucose level (%) | 27.4 | 19.5 | 31.5 |
| Dyslipidemia (%) | 33.4 | 15.9 | 42.6 |

Fig. 5 shows correlation diagrams of the total adiponectin (Total-APN) amount, high-molecular-weight adiponectin (HMW-APN) amount, C1q-adiponectin complex (C1q-APN) amount, and C1q amount in the circulating blood. In Fig. 5, open circles indicate the group of VFA ≥ 100 cm², and closed circles indicate the group of VFA < 100 cm².

The Total-APN amount showed a significant positive correlation with the HMW-APN amount (r = 0.9518, p < 0.0001; Fig. 5A), and showed a dispersed positive correlation with the C1q-APN amount (r= 0.5657, p < 0.0001; Fig. 5B). On the other hand, no correlation was found between the Total-APN amount and the C1q amount (Fig. 5D).

The HMW-APN amount showed a significant positive correlation with the C1q-APN amount (r = 0.5577, p < 0.0001; Fig. 5C), but did not show a correlation with the C1q amount (Fig. 5E). In contrast, the C1q amount showed a weak correlation with the C1q-adiponectin complex amount (r = 0.1140, p = 0.0411; Fig. 5F).

Next, the correlation between the C1q-APN amount or the C1q amount, and various clinical parameters was analyzed. Table 2 shows the correlation coefficients. Since the IRI, TG, ALT, γ-GTP, and C1q-APN amounts showed asymmetric distribution, they were subjected to log conversion before being used in the analysis. Log C1q-APN was positively correlated with HDL-C, and negatively correlated with BMI, WC, VFA, SFA, log-IRI, HbA1c, log-TG, UA, log-ALT, and log-γ-GTP. On the other hand, no correlation was found between Log C1q-APN and the number of risk factors. C1q showed a positive correlation with BMI, WC, VFA, SFA, SBP, DBP, log IRI, TC, log TG, Log ALT, and the number of risk factors, and showed a negative correlation with HDL-C.

Further, stepwise multiple regression analysis showed that BMI (multivariate BMI), VFA (multivariate VFA), and SFA (multivariate SFA) were significant regulation factors of the C1q-APN complex amount. In contrast, BMI, VFA, and SFA were not significant regulation factors of C1q.

In Table 2, the data for levels of C1q-APN, IRI, TG, AST, ALT, and γ-GTP showed strain distribution, and was logarithmically transformed before analysis. The significance level was set at p < 0.05 (*). Stepwise multiple regression analysis was performed, and these parameters significantly correlated with C1q-APN or C1q were determined. Subsequently, the parameters of p < 0.05 and F > 4.0 were applied to regression analysis as independent variables (*).

These abbreviations are the same as those in Table 1.

**Table 2.A**

| | Log C1Q-AN | | | | |
|---|---|---|---|---|---|
| | Univariate | | Multivariate | | |
| | r | p value | BMI | VFA | SFA |
| | | | F value | | |
| Age | 0.1049 | 0.0545 | | | |
| BMI | -0.2366 | <0.0001* | 8.308* | ------ | ------ |
| WC | -0.2258 | <0.0001* | | | |
| VFA | -0.2191 | <0.0001* | ----- | 6.697* | ------ |
| SFA | -0.1761 | 0.0013* | ----- | ----- | 5.334* |
| SBP | 0.0000 | >0.9999 | | | |
| DBP | -0.0032 | 0.9545 | | | |
| Log IRI | -0.1612 | 0.0034* | 0.132 | 0.674 | 0.577 |
| Glucose | -0.0894 | 0.0969 | | | |
| HbA1c | -0.1517 | 0.0057* | 5.177* | 4.469* | 7.114* |
| TC | -0.0200 | 0.7174 | | | |
| Log Triglyceride | -0.1612 | 0.0034* | 1.104 | 2.171 | 1.412 |
| HDL-C | 0.1517 | 0.0055* | 3.168 | 2.441 | 3.782 |
| UA | -0.2121 | 0.0001* | 8.149* | 11.047* | 10.179* |
| Cre | -0.1000 | 0.0648 | | | |
| eGFR | 0.0548 | 0.3424 | | | |
| Log AST | -0.0316 | 0.6056 | | | |
| Log ALT | -0.1703 | 0.0018* | 0.220 | 1.300 | 0 |
| Log γ-GTP | -0.1897 | 0.0006* | 4.659* | 3.939 | 5.053* |
| Brinkman index | -0.0447 | 0.4744 | | | |
| Number of risk factors | -0.0837 | 0.1219 | | | |
| Correction r² | | | 0.100 | 0.080 | 0.092 |

**Table 2.B**

| | C1q | | | | |
|---|---|---|---|---|---|
| | Univariate | | Multivariate | | |
| | r | p value | BMI | VFA | SFA |
| | | | F value | | |
| Age | 0.0054 | 0.9222 | | | |
| BMI | 0.1732 | 0.0017* | 0.421 | ------ | ------ |
| WC | 0.1517 | 0.0054* | | | |
| VFA | 0.1449 | 0.0093* | ------ | 0.219 | ------ |
| SFA | 0.1265 | 0.0237* | ------ | ------ | 0.007 |
| SBP | 0.1897 | 0.0005* | 6.455* | 8.607* | 8.607* |
| DBP | 0.1871 | 0.0007* | 0.635 | 0.595 | 0.595 |
| Log IRI | 0.2000 | 0.0003* | 6.605* | 5.124* | 5.124* |
| Glucose | 0.0447 | 0.4112 | | | |
| HbA1c | -0.0028 | 0.9599 | | | |
| TC | 0.1378 | 0.0128* | 1.311 | 1.267 | 1.267 |
| Log Triglyceride | 0.2074 | 0.0001* | 7.555* | 8.578* | 8.578* |
| HDL-C | -0.1871 | 0.0006* | 1.972 | 2.312 | 2.312 |
| UA | 0.0316 | 0.6462 | | | |
| Cre | 0.0447 | 0.4823 | | | |
| eGFR | -0.0447 | 0.4272 | | | |
| Log AST | 0.0837 | 0.1408 | | | |
| Log ALT | 0.1140 | 0.0381* | 0.000 | 0.032 | 0.032 |
| Log γ-GTP | 0.0046 | 0.9338 | | | |
| Brinkman index | -0.0447 | 0.3894 | | | |
| Number of risk factors | 0.1761 | 0.0013* | 0.224 | 0.062 | 0.062 |
| Correction r² | | | 0.077 | 0.084 | 0.084 |

### Example 7: ROC Curve Analysis with Respect to Determination of Presence of Metabolic Syndrome

The total adiponectin (Total-APN) amount in the circulating blood is known to be a biomarker for metabolic syndrome (NPL 7). Not only the absolute amount, but also a relative indicator showing the structure of adiponectin in the blood, such as the abundance of high-molecular-weight adiponectin (HMW-APN) in Total-APN (HMW-APN/Total-APN ratio) or the abundance of C1q-adiponectin complexes in the total adiponectin amount (C1q-APN/Total-APN ratio), was also considered important for observing qualitative changes. The relationship between the C1q-APN amount, C1q amount, C1q-APN/Total-APN ratio, HMW-APN/Total-APN ratio, or C1q-APN/C1q ratio in the circulating blood and metabolic syndrome was completely unknown.

Table 3 shows ROC curves of various clinical indicators for the determination of the presence of metabolic syndrome when subjects with visceral fat accumulation were defined by VFA ≥ 100 cm² or WC ≥ 85 cm. When the visceral fat accumulation group was defined by VFA ≥ 100 cm² (Fig. 6, Table 3), the ROC area under the specificity-sensitivity curve (AUC) of each indicator was as follows:
C1q-APN/Total-APN ratio: 0.704 (95%CI: 0.632-0.775; Fig. 6E)
Total-APN amount: 0.668 (95%CI: 0.596-0.740; Fig. 6A)
HMW-APN amount: 0.648 (95%CI: 0.577-0.720; Fig. 6B)
C1q-APN amount: 0.535 (95%CI: 0.457-0.613; Fig. 6C)

Regarding these results, the same results were obtained when metabolic syndrome was defined by WC (Table 3). Table 3 is ordered by AUC, from highest. The abbreviations in Table 3 are as follows.
AUC: specificity-sensitivity area under curve
95%CI: 95% confidence interval
Other abbreviations are the same as those in Table 1.

**Table 3.A**

| | MetS (VFA ≥100 cm²) | | | |
|---|---|---|---|---|
| | AUC | 95% CI | | P value |
| | | Lower limit | Upper limit | |
| BG | 0.830 | 0.772 | 0.888 | <0.0001 |
| VFA | 0.813 | 0.760 | 0.866 | <0.0001 |
| SBP | 0.800 | 0.731 | 0.868 | <0.0001 |
| Triglyceride | 0.760 | 0.694 | 0.827 | 0.0002 |
| DBP | 0.739 | 0.666 | 0.812 | <0.0001 |
| IRI | 0.733 | 0.663 | 0.802 | <0.0001 |
| BMI | 0.712 | 0.639 | 0.784 | <0.0001 |
| WC | 0.712 | 0.641 | 0.782 | <0.0001 |
| C1q-APN/Total-APN | 0.703 | 0.632 | 0.775 | <0.0001 |
| C1q/Total-APN | 0.695 | 0.626 | 0.764 | <0.0001 |
| C1q/HMW-APN | 0.675 | 0.607 | 0.743 | 0.0029 |
| C1q-APN/HMW-APN | 0.673 | 0.602 | 0.743 | 0.0022 |
| Total-APN | 0.668 | 0.596 | 0.740 | 0.0002 |
| HDL-C | 0.662 | 0.582 | 0.742 | 0.0005 |
| HbA1c | 0.658 | 0.579 | 0.738 | 0.0023 |
| γ-GTP | 0.656 | 0.584 | 0.729 | 0.0214 |
| HMW-APN | 0.648 | 0.577 | 0.720 | 0.0008 |
| ALT | 0.633 | 0.550 | 0.715 | 0.0174 |
| Age | 0.630 | 0.552 | 0.708 | 0.0040 |
| SFA | 0.630 | 0.556 | 0.704 | 0.0061 |
| Brinkman index | 0.622 | 0.538 | 0.707 | 0.0020 |
| C1q | 0.615 | 0.541 | 0.688 | 0.0039 |
| C1q-APN/C1q | 0.600 | 0.525 | 0.675 | 0.0122 |
| TC | 0.597 | 0.520 | 0.674 | 0.0172 |
| AST | 0.596 | 0.510 | 0.682 | 0.0801 |
| UA | 0.594 | 0.515 | 0.673 | 0.0328 |
| eGFR | 0.590 | 0.511 | 0.668 | 0.0245 |
| HMW/Total-APN | 0.580 | 0.507 | 0.652 | 0.0499 |
| Cr | 0.550 | 0.468 | 0.633 | 0.1838 |
| C1q-APN | 0.535 | 0.457 | 0.613 | 0.2249 |

**Table 3.B**

| | MetS (WC ≥85 cm) | | | |
|---|---|---|---|---|
| | AUC | 95% CI | | P value |
| | | Lower limit | Upper limit | |
| WC | 0.876 | 0.835 | 0.918 | <0.0001 |
| VFA | 0.876 | 0.822 | 0.929 | <0.0001 |
| BMI | 0.838 | 0.779 | 0.896 | <0.0001 |
| BG | 0.829 | 0.767 | 0.891 | <0.0001 |
| IRI | 0.790 | 0.716 | 0.864 | <0.0001 |
| SBP | 0.760 | 0.675 | 0.845 | <0.0001 |
| C1q-APN/Total-APN | 0.757 | 0.679 | 0.835 | <0.0001 |
| SFA | 0.748 | 0.677 | 0.819 | <0.0001 |
| DBP | 0.743 | 0.659 | 0.827 | <0.0001 |
| C1q/Total-APN | 0.729 | 0.647 | 0.810 | <0.0001 |
| Triglyceride | 0.717 | 0.638 | 0.797 | 0.0071 |
| C1q-APN/HMW-APN | 0.716 | 0.638 | 0.794 | 0.0003 |
| C1q/HMW-APN | 0.704 | 0.624 | 0.783 | 0.0005 |
| Total-APN | 0.703 | 0.619 | 0.787 | 0.0002 |
| ALT | 0.700 | 0.610 | 0.789 | 0.0045 |
| HbA1c | 0.694 | 0.605 | 0.784 | 0.0015 |
| HDL-C | 0.687 | 0.596 | 0.777 | 0.0009 |
| HMW-APN | 0.679 | 0.596 | 0.761 | 0.0015 |
| AST | 0.648 | 0.549 | 0.747 | 0.0197 |
| γ-GTP | 0.631 | 0.544 | 0.718 | 0.0632 |
| C1q | 0.626 | 0.538 | 0.714 | 0.0056 |
| eGFR | 0.618 | 0.531 | 0.704 | 0.0202 |
| UA | 0.617 | 0.527 | 0.707 | 0.0376 |
| TC | 0.608 | 0.518 | 0.697 | 0.0449 |
| Age | 0.605 | 0.509 | 0.701 | 0.0612 |
| C1q-APN/C1q | 0.594 | 0.501 | 0.686 | 0.0493 |
| HMW-APN/Total-APN | 0.592 | 0.507 | 0.676 | 0.0519 |
| Cr | 0.588 | 0.496 | 0.679 | 0.1010 |
| Brinkman index | 0.568 | 0.467 | 0.668 | 0.1498 |
| C1q-APN | 0.521 | 0.427 | 0.615 | 0.6018 |

The above results revealed the possibility that, among the adiponectin-related indicators, the C1q-APN/Total-APN ratio was the most effective biomarker for the determination of metabolic syndrome, compared to the Total-APN amount or the HMW-APN amount. In contrast, the amount of C1q-APN alone was not an effective indicator.

### Example 8: Various Types of Adiponectin in Visceral Fat Accumulation and Obesity-Related Cardiovascular Risk Factor Accumulation

A group having dyslipidemia, hypertension, and/or abnormality in blood sugar (component factors of metabolic syndrome) was evaluated on the relevance to various adiponectin-related indicators. The subjects were classified into two groups: VFA < 100 cm² and VFA ≥ 100 cm². Cutoff values were selected on the basis of the Japanese visceral fat accumulation standard. Table 1 shows the clinical characteristics of these two groups.

In the group of VFA < 100 cm², no relevance was observed between the obesity-related cardiovascular risk factor accumulation, and the Total-APN amount, HMW-APN amount, C1q-APN amount, HMW-APN/Total-APN ratio, C1q-APN/Total-APN ratio, or C1q-APN/HMW-APN ratio in the serum (Fig. 7A). Comparatively, in the group of VFA ≥ 100 cm², the Total-APN amount and HMW-APN amount in the serum showed a significant negative correlation with obesity-related cardiovascular risk factor accumulation (p = 0.0191 (Fig. 7B a), p = 0.0450 (Fig. 7Bb); Kruskal-Wallis test). The C1q amount showed a significant positive correlation with risk factor accumulation (p = 0.0191 (Fig. 7Bd)), but showed no correlation with C1q-APN (p = 0.5669 (Fig. 7B d)). Further, the C1q-APN/Total-APN ratio and C1q-APN/HMW-APN ratio showed a significant positive correlation with obesity-related cardiovascular risk factor accumulation (p = 0.0003 (Fig. 7B e), p = 0.0065 (Fig. 7B f); Kruskal-Wallis test); whereas the HMW-APN/Total-APN ratio and C1q-APN/C1q ratio did not show a correlation with risk-factor accumulation (Figs. 7Bg, h).

The above results revealed that, compared to other indicators, the C1q-APN/Total-APN ratio is a biomarker that most highly reflects obesity-related cardiovascular risk factor accumulation. This strongly suggests the possibility that the present indicator is associated with arteriosclerosis.

## Claims

1. A C1q-adiponectin complex comprising C1q and naturally occurring adiponectin.

2. A method for measuring the amount of the Clq-adiponectin complex according to claim 1, the method comprising measuring the amount of the complex by an immunoassay.

3. The measurement method according to claim 2, wherein the immunoassay is an ELISA method.

4. The measurement method according to claim 3, wherein the ELISA method is a sandwich ELISA method using an anti-adiponectin antibody and an anti-C1q antibody.

5. The measurement method according to claim 4, wherein the anti-adiponectin antibody is F(ab')2.

6. A method for diagnosing metabolic syndrome or arteriosclerosis risk, the method comprising measuring the amount of the C1q-adiponectin complex according to claim 1 in blood taken from a diagnosis target, by the measurement method according to any one of claims 2 to 5.

7. The diagnosis method according to claim 6, which uses, as an indicator, a ratio of the amount of the C1q-adiponectin complex according to claim 1 to the total adiponectin amount (C1q-APN/Total-APN ratio) in the blood.

8. A kit for measuring the amount of the C1q-adiponectin complex according to claim 1, the kit comprising an anti-adiponectin antibody and an anti-C1q antibody.

9. The kit according to claim 8, which is for diagnosis of metabolic syndrome or arteriosclerosis risk.
